# EUROPEAN PATENT APPLICATION

(11) **EP 4 432 147 A1**
(43) Date of publication of application: **18.09.2024**
(21) Application number: 24162844.5
(22) Date of filing: 12.03.2024
(51) Int. Cl.: G06F 21/56, G16H 30/20, G16H 30/40

(54) **SYSTEM AND METHODS FOR ANOMALY AND MALWARE DETECTION IN MEDICAL IMAGING DATA**

(30) Priority: 17.03.2023 US 202318122897
(71) Applicant: Optum, Inc., Minnetonka, Minnesota 55343 (US)
(72) Inventor: CARLETON, Jesse, Victoria, V9A 1P6 (CA)
(74) Representative: Zimmermann & Partner Patentanwälte mbB

(57) **Abstract**

A system and methods for detecting anomalies and/or malware in medical imaging files are disclosed. The method generally includes obtaining a medical imaging file, evaluating the medical imaging file using a classification model to determine a first score representative of a likelihood that the medical imaging file contains anomalous or malicious data and to identify suspected anomalous or malicious data within the medical imaging file, modifying the medical imaging file that removes suspected anomalous or malicious data from the medical imaging file if the first score meets or exceeds a threshold, and if the first score is less than the first threshold, at least one of: i) store the medical imaging file without said modification, or ii) retrain the classification model based on the medical imaging file.

## Description

### BACKGROUND

Medical imaging generally refers to the use of medical imaging technologies to capture images of a subject's body for diagnosing, monitoring, and/or treatment of various conditions and ailments. Medical imaging data is often captured by a medical imaging device (e.g., ultrasound, X-ray, magnetic resonance imaging (MRI), etc.) and communicated to an external computing device(s) for processing and/or storage. For example, medical imaging data may be communicated to a picture archiving and communication system (PACS). Users (e.g., medical professionals) can then remotely access stored medical imaging data from a workstation or other computing device. In many cases, medical imaging data is communicated via the Digital Imaging and Communications in Medicine (DICOM) standard, which defines a data interchange protocol, file format, and structure for medical images and image-related metadata.

Not unlike other types of computer files, DICOM files are susceptible to malware, ransomware, and other types of cybersecurity attacks. Accessing a DICOM file via a remote workstation, for example, risks spreading malicious data between systems. Traditional antivirus and antimalware software may not be suitable for medical imaging applications, as many existing techniques default to quarantining or deleting files which would impact the availability of studies. Even assuming that existing antivirus and antimalware solutions were capable of detecting malicious code in DICOM files, deploying DICOM files to a third-party system for virus/malware detection would create unacceptable latency and delay in accessing medical images (e.g., in critical care settings). Further, there is a lack of visibility and capability for detecting anomalies in DICOM data. Anomalies can indicate that a file has been compromised or subject to a hacking attempt but, in some cases, non-malicious activities can also cause anomalies which are not necessarily harmful. For example, new modalities (e.g., medical imaging systems) can generate DICOM payloads in a new or unexpected manner, which can be a challenge to differentiate from malicious behavior.

### SUMMARY

One implementation of the present disclosure is a system for detecting anomalies and malware in medical imaging data. The system includes a processor and memory having instructions stored thereon that, when executed by the processor, cause the system to: obtain a medical imaging file including a header and a data set, where the header includes metadata associated with the medical imaging file and the data set includes images captured by a medical imaging device; evaluate the medical imaging file using a classification model to: i) determine a first score representative of a likelihood that the medical imaging file contains anomalous or malicious data, and ii) identify suspected anomalous or malicious data within the medical imaging file; if the score meets or exceeds a first threshold, generate a modified version of the medical imaging file by removing the suspected anomalous or malicious data from the medical imaging file while retaining valid metadata in the header and the images in the data set; and if the first score is less than the first threshold, at least one of: i) store the medical imaging file without said modification, or ii) retrain the classification model based on the medical imaging file.

In some implementations, the instructions further cause the system to evaluate the modified medical imaging file using the classification model to generate a second score representative of a likelihood that the modified medical imaging file contains anomalous or malicious data and, if the second score meets or exceeds the first threshold, quarantine the medical imaging file or flag the medical imaging file for additional review.

In some implementations, the instructions further cause the system to, if the first score is between the first threshold and the second threshold, modify or retrain the classification model using the medical imaging file without generating the modified version of the medical imaging file.

In some implementations, the medical imaging file is stored without modification if the first score is less than a second threshold, wherein the second threshold is lower than the first threshold.

In some implementations, the medical imaging file is used to retrain the classification model if the first score is between the first threshold and a second threshold, wherein the second threshold is lower than the first threshold.

In some implementations, the processor and the memory are components of an edge server of a picture archiving and communication system (PACS), and the medical imaging file is received by the edge server from the medical imaging device.

In some implementations, the instructions further cause the system to convert the images in the data set of the medical imaging file to greyscale prior to evaluating the medical imaging file using the classification model.

In some implementations, the medical imaging file is a DICOM file.

In some implementations, the classification model is one of a multi-layer perceptron (MLP) model, a support vector machine (SVM) model, random forest model, or a convolutional neural network (CNN).

In some implementations, the classification model is a first classification model and the first score is representative of a likelihood that the medical imaging file contains an anomaly, the instructions further causing the system to evaluate the medical imaging file using a second classification model that generates a second score representative of a likelihood that the medical imaging file contains malware.

Another implementation of the present disclosure is a method for detecting anomalies and malware in medical imaging data. The method includes: obtaining a medical imaging file including a header and a data set, where the header includes metadata associated with the medical imaging file and the data set includes images captured by a medical imaging device; evaluating the medical imaging file using a classification model to: i) determine a first score representative of a likelihood that the medical imaging file contains anomalous or malicious data, and ii) identify suspected anomalous or malicious data within the medical imaging file; if the score meets or exceeds a first threshold, generating a modified version of the medical imaging file by removing the suspected anomalous or malicious data from the medical imaging file while retaining valid metadata in the header and the images in the data set; and if the first score is less than the first threshold, at least one of: i) storing the medical imaging file without said modification, or ii) retraining the classification model based on the medical imaging file.

In some implementations, the method further includes evaluating the modified medical imaging file using the classification model to generate a second score representative of a likelihood that the modified medical imaging file contains anomalous or malicious data and, if the second score meets or exceeds the first threshold, quarantining the medical imaging file or flagging the medical imaging file for additional review.

In some implementations, the medical imaging file is stored without modification if the first score is less than a second threshold, wherein the second threshold is lower than the first threshold.

In some implementations, the medical imaging file is used to retrain the classification model if the first score is between the first threshold and a second threshold, wherein the second threshold is lower than the first threshold.

In some implementations, the classification model is trained by a remote computing device using a training set of known good medical imaging data, the method further including receiving the classification model from the remote device after training.

In some implementations, the medical imaging file is obtained by an edge server of a picture archiving and communication system (PACS) and from the medical imaging device.

In some implementations, the method further includes converting the images in the data set of the medical imaging file to greyscale prior to evaluating the medical imaging file using the classification model.

In some implementations, the medical imaging file is a DICOM file.

In some implementations, the classification model is one of a multi-layer perceptron (MLP) model, a support vector machine (SVM) model, random forest model, or a convolutional neural network (CNN).

Yet another implementation of the present disclosure is a non-transitory computer readable medium having instructions stored thereon that, when executed by a processor, cause a device to: obtain a medical imaging file including a header and a data set, where the header includes metadata associated with the medical imaging file and the data set includes images captured by a medical imaging device; evaluate the medical imaging file using a classification model to: i) determine a first score representative of a likelihood that the medical imaging file contains anomalous or malicious data, and ii) identify suspected anomalous or malicious data within the medical imaging file; and compare the first score to a first threshold, wherein: if the first score meets or exceeds the first threshold, then the instructions further cause the device to modify the medical imaging file by removing the suspected anomalous or malicious data from the medical imaging file while retaining valid metadata in the header and the images in the data set; or if the first score is less than the first threshold, then the instructions further cause the device to at least one of: i) store the medical imaging file without said modification, or ii) retrain the classification model based on the medical imaging file.

Additional features will be set forth in part in the description which follows or may be learned by practice. The various features described herein will be realized and attained by means of the elements and combinations particularly pointed out in the appended claims. It is to be understood that both the foregoing general description and the following detailed description are exemplary and explanatory only and are not restrictive, as claimed.

### BRIEF DESCRIPTION OF THE DRAWINGS

FIG. 1 is a block diagram of an example medical image processing architecture, according to some implementations.
FIG. 2 is a block diagram of a payload classification system for detecting and remedying anomalies and malware in medical imaging data, according to some implementations.
FIG. 3 is a flow chart of a process for detecting anomalies in medical imaging data, according to some implementations.
FIG. 4 is a flow chart of a process for detecting malware in medical imaging data, according to some implementations.
FIG. 5 is a flow chart of a process for content disarm and reconstruction of medical imaging data, according to some implementations.

Various objects, aspects, and features of the disclosure will become more apparent and better understood by referring to the detailed description taken in conjunction with the accompanying drawings, in which like reference characters identify corresponding elements throughout. In the drawings, like reference numbers generally indicate identical, functionally similar, and/or structurally similar elements.

### DETAILED DESCRIPTION

Referring generally to the figures, a system and methods for detecting and remedying anomalies and malware in medical imaging data are shown, according to various implementations. "Medical imaging data," as referred to herein, generally refers to one or more medical imaging files containing images captured by medical imaging equipment. Often, medical imaging data is formatted according to DICOM standards; thus, "medical imaging data" or "medical imaging files" - used interchangeably herein - generally refers to, but is not limited to, DICOM files. A medical imaging file (e.g., a DICOM file) generally includes a header containing metadata associated with the medical images and a data set containing the images themselves. In a DICOM file, in particular, the header may include a preamble and a prefix, and the data set may contain a plurality of data elements.

An "anomaly" generally refers to any anomalous (e.g., out-of-the-ordinary or unexpected) data in a medical imaging file, as would be generally understood by those of ordinary skill in the art. Anomalies can include, for example, new or unrecognized metadata, new or unrecognized data formats, broken files or data, and the like. In some cases, anomalies are malicious. For example, malicious code that is embedded into a DICOM file may be considered an anomaly. However, not all anomalies are malicious in nature. For example, a new medical imaging device (e.g., modality) may generate DICOM files that are different, or that contain different data, than an existing device, which may initially appear anomalous; yet the DICOM files generated by the new medical imaging device are not necessarily harmful or malicious. Thus, the system and methods described herein are configured in part to differentiate harmful and non-harmful anomalies.

"Malware" generally refers to malicious code or data intended to infiltrate, disrupt, or otherwise harm a computing device or system, as would be generally understood by those of ordinary skill in the art. Accordingly, as used herein, it should be understood that "malware" can also refer to viruses, ransomware, and the like. As mentioned above, medical imaging data (e.g., DICOM files) is often generated by a medical imaging device and communicated to one or more external systems (e.g., a PACS); thus, malware-infected files can easily propagate through a network or system, infecting multiple devices. Using a traditional antivirus or antimalware software to scan DICOM files may result in the files being indiscriminately quarantined or deleted, which would impact availability of studies for end users (e.g., medical professionals). In many cases, antivirus or antimalware software is also not designed to evaluate image data and/or the specific formatting of medical imaging files. Additionally, existing antivirus and antimalware techniques may not comply with privacy and regulatory requirements (e.g., FDA certifications, HIPAA, etc.) and can require medical imaging data to be transmitted to third-party systems which introduces significant implementation challenges and processing delays.

At a high level, according to implementations of the present disclosure, medical imaging (e.g., DICOM) files are obtained and evaluated using trained classification models, which classify the files as either "likely to contain" or "not likely to contain" anomalies and/or malware. In some implementations, the trained classification models also output scores for the classifications, which may be indicative of a likelihood that the medical imaging data contains anomalies or malware, respectively. In some cases, separate classification models are trained for anomaly and malware detection using corresponding training data sets. For example, the medical imaging data can be evaluated by a first classification model for detecting anomalies, which outputs a prediction of whether the medical imaging data contains an anomaly and/or a confidence score for the prediction. In some implementations, the first classification model is trained using a training data set of known-good medical imaging files (e.g., DICOM files) using a variety of modalities, attachments, embedded objects, etc. Similarly, medical imaging data can be evaluated by a second classification model for detecting malware, which outputs a prediction of whether the medical imaging data contains a malware and/or a confidence score for the prediction. In some implementations, the second classification model is trained using a training data set of known-malicious files and malware samples.

In some implementations, if a medical imaging file is classified as "likely to contain" anomalies or malware, and/or the confidence score of the classification is above a threshold value, the medical imaging file may be modified to remove identified and possibly malicious data elements. In some such implementations, the medical imaging file is passed to a "content disarm and reassembly" subsystem which disassembles the file, removes identified and possibly malicious data elements, and reassembles the file in a safe state. The modified (e.g., reassembled) file may then be reevaluated by the classification models to determine whether, after removing suspected malicious elements, the medical imaging file is still "likely to contain" anomalies and/or malware. If so, the medical imaging file may be flagged for manual review and/or corrective actions may be initiated. Further details of the disclosed anomaly and malware detection system and methods are described in detail below.

### Overview

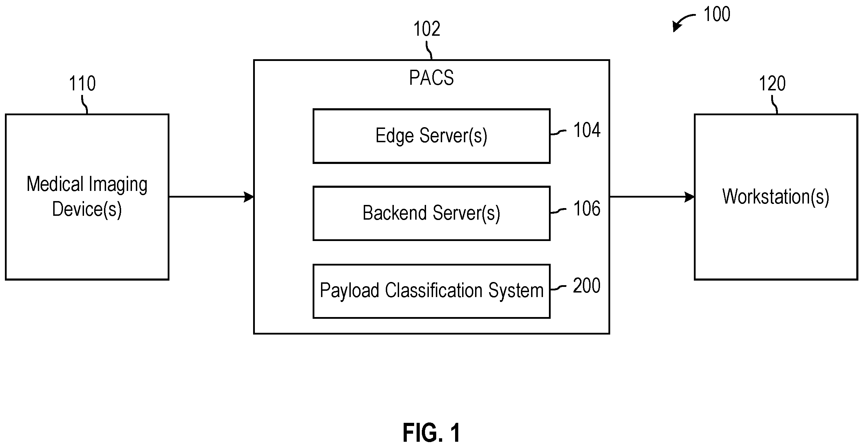

Referring now to FIG. 1, a block diagram of an example medical image processing architecture 100 is shown, according to some implementations. Broadly, architecture 100 includes a picture archiving and communication system (PACS) 102, medical imaging device(s) 110, and workstation(s) 120. In some implementations, medical imaging device(s) 110 and/or workstation(s) 120 can be consider a part of PACS 102; however, for clarity, medical imaging device(s) 110 and workstation(s) 120 are described separately herein. Medical imaging device(s) 110 may include any number and type of medical imaging device - also referred to as "imaging modalities." For example, medical imaging device(s) 110 may include one or more ultrasound devices, X-ray machines, MRI machines, computed tomography (CT) machines, and the like. It should be appreciated that the present description is not limited to a particular type of medical imaging device or a particular number of medical imaging devices. Further, it should be appreciated that medical imaging device(s) 110 may include multiple different types of imaging modalities.

Workstation(s) 120 may include any number of remote computing devices from which medical imaging data can be viewed and/or manipulated. More specifically, workstation(s) 120 may be computing devices that can communicate with PACS 102 to retrieve medical imaging data for viewing. Workstation(s) 120 can, accordingly, include any number and type of computers, servers, processing units, and the like. For example, workstation(s) 120 may be desktop and/or laptop computers that include a display screen, through which a user can access PACS 102 remotely (e.g., via the Internet). As another example, workstation(s) 120 may be any computing device that executes medical image viewing software (e.g., a DICOM viewer). It should be appreciated that workstation(s) 120 is not limited to a particular type of computing device and that workstation(s) 120 may include multiple different types of computing devices.

PACS 102 is generally a system of one or more interconnected computing devices that store and optionally process and/or manipulate medical imaging data. For example, PACS 102 can receive medical imaging files from medical imaging device(s) 110 and can store the medical imaging files for later retrieval and/or viewing by one or more of workstation(s) 120. In some implementations, PACS 102 includes one or more edge server(s) 104 and/or backend server(s) 106. Edge server(s) 104 and/or backend server(s) 106 can each include one or more computing devices configured to perform appropriate functions with respect to the processing and/or storage of medical images, as described in greater detail below. It should be understood that, in various implementations, PACS 102 can be configured without one or more of edge server(s) 104 and/or backend server(s) 106, and/or can include additional components not described herein. It should also be appreciated that PACS 102 can include any number of edge server(s) 104 and/or backend server(s) 106.

Edge server(s) 104 are generally configured to receive medical imaging data (e.g., DICOM files) from medical imaging device(s) 110 for processing and/or storage. In some implementations, medical imaging data is transmitted directly to edge server(s) 104 from medical imaging device(s) 110, e.g., after capturing the images and/or generating a medical image file. In some such implementations, edge server(s) 104 are in direct communication with medical imaging device(s) 110 (e.g., via a network or a direct connection). In some implementations, medical imaging device(s) 110 may first transmit medical imaging files to an additional gateway device (e.g., another computing device) which routes the medical imaging file to an appropriate one of edge server(s) 104. In some implementations, edge server(s) 104 are also in communication with workstation(s) 120 (e.g., via a network) to facilitate the retrieval and/or viewing of medical imaging data. For example, workstation(s) 120 may interact with edge server(s) 104 via the Internet or a private network in order to view images and image-related metadata. In some implementations, edge server(s) 104 retain medical imaging data for a set amount of time (e.g., a day, a week, etc.), such that the medical imaging data can be quickly accessed via workstation(s) 120. In some implementations, edge server(s) 104 retain medical imaging data indefinitely.

In some implementations, edge server(s) 104 are distributed servers or other computing devices that are located at various different geographical locations. For example, edge server(s) 104 may serve a single medical facility or a group of medical facilities (e.g., within a region or a company). Thus, edge server(s) 104 may be physically located in proximity to the one or more medical facilities. As an example, a medical facility may have its own edge server 104 for locally storing medical imaging data, or an edge server 104 may serve a group of medical facilities within a common geographical region. In some implementations, in addition to storing medical imaging data, edge server(s) 104 are configured to process the medical imaging data, such as by checking and/or editing patient demographic information and other attributes of a study. In some implementations, edge server(s) 104 validate and/or edit the metadata of a medical imaging file, which can include said patient demographic information and study attributes. A "study," as mentioned herein, generally refers to a set of one or more medical imaging files (e.g., DICOM files) relating to a test sequence or procedure for a particular patient. For example, a study may include multiple views of a part of the patient's body.

Backend server(s) 106 are generally configured to archive medical imaging data. To this point, backend server(s) 106 may, in some cases, have a larger storage capacity and/or greater processing capabilities than edge server(s) 104. In some implementations, backend server(s) 106 include one or more computing devices configured for long-term data storage. In this manner, edge server(s) 104 may transmit medical imaging files or copies of medical imaging files to backend server(s) 106 for storage. In some implementations, medical imaging files can be later retrieved from backend server(s) 106 by workstation(s) 120, either directly or through edge server(s) 104. For example, if a user of a workstation requests an archived study which is no longer retained on an edge server, the edge server may forward the request to backend server(s) 106 and/or may retrieve the archived study for viewing. In some implementations, backend server(s) 106 are located remotely from edge server(s) 104, such as at or near a facility associated with an entity that operates PACS 102 or provides PACS services.

In addition to archiving data, backend server(s) 106 may handle various operational and/or management tasks for PACS 102 and/or edge server(s) 104. In some implementations, for example, backend server(s) 106 can provide software and firmware updates to edge server(s) 104. In some implementations, backend server(s) 106 are configured to generate and/or train classification models which are distributed to edge server(s) 104 for detecting anomalies and/or malware. The classification models discussed herein and associated training techniques are described in greater detail below but, at a high level, backend server(s) 106 may train the classification models for one or both of anomaly and malware detection using suitable training data sets. In this regard, backend server(s) 106 may also generate, maintain, and/or retrieve training data for anomaly and/or malware detection. With respect to anomaly detection, for example, backend server(s) 106 may generate and/or maintain a data set of known-good imaging data from medical imaging device(s) 110. In some implementations, backend server(s) 106 can augment the training data set as additional medical imaging data is received. With respect to malware detection, backend server(s) 106 may maintain and/or retrieve a data set of known malicious code/malware. In some implementations, a malware training data set can be maintained in a secure environment within backend server(s) 106. In other implementations, backend server(s) 106 may retrieve malware training data from external sources.

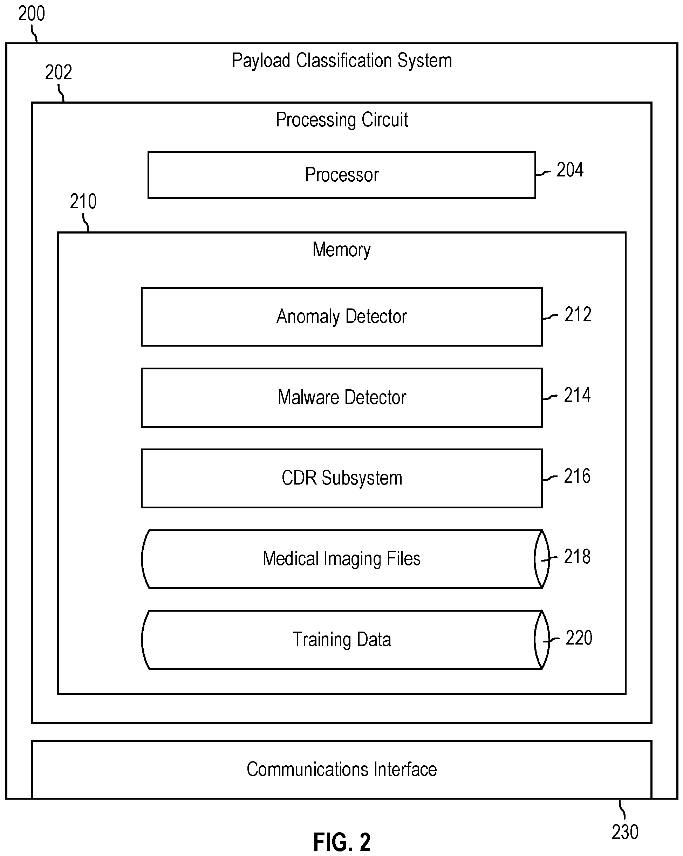

While not explicitly illustrated in FIG. 1, it should be appreciated that the various components of architecture 100 (e.g., PACS 102, medical imaging device(s) 110, and workstation(s) 120) may communicate (e.g., transmit and receive data) via any suitable method or structure. In some implementations, PACS 102, medical imaging device(s) 110, and/or workstation(s) 120 communicate via a network or multiple different types of networks, such as a local area network (LAN), a wide area network (WAN), a virtual private network (VPN), or the like. In some implementations, communications between one or more components of architecture 100 are direct (e.g., without a network). For example, medical imaging device(s) 110 may be directly coupled to an edge server 104 for communicating medical imaging data or may communicate with an edge server 104 via a suitable network. Accordingly, each component may include suitable wired and/or wireless communication interface(s), as described in greater detail below with respect to FIG. 2. In some implementations, each component of architecture 100 includes a suitable web-based interface for network communications. It should be appreciated that the present disclosure is not intended to be limited to any specific communication or network structure.

As shown, PACS 102 further includes a payload classification system 200, which is generally configured to implement the anomaly and malware detection techniques described herein. In particular, as described in greater detail below with respect to FIG. 2, payload classification system 200 may evaluate received medical imaging data (e.g., DICOM files) using trained anomaly and/or malware detection models (e.g., classification models) to generate predictions of whether a medical imaging file contains anomalies and/or malware. If it is predicted that a file likely contains anomalies and/or malware, payload classification system 200 may execute a content disarm and reconstruction process to disassemble the file, remove any potentially anomalous and/or malicious elements, and reassemble the file in a secure environment. Payload classification system 200 may then reevaluate the file to determine whether the file still contains anomalies and/or malware after content disarm and reconstruction. If so, corrective actions may be initiated (e.g., the file flagged for manual review); if not, processing of the file may continue as normal. Similarly, medical imaging files that are predicted not to be malicious or to contain anomalies, initially, may be processed as normal (e.g., without content disarm and reconstruction).

For added clarity, consider the following example use-case of architecture 100. In this example, medical imaging device(s) 110 include an X-ray machine that captures a series of images of a patient for a first study at a first medical facility. From these captured images, the X-ray machine may generate one or more DICOM files which are transmitted to edge server(s) 104 for initial processing and/or storage. In this example, edge server(s) 104 can include a first edge server that is local to the first medical facility (e.g., that is physically hosted by the first medical facility). Upon receipt, the first edge server can invoke payload classification system 200 to evaluate the DICOM file(s) for anomalies and/or malware. For anomaly detection, payload classification system 200 may execute an anomaly detection model that determines whether the file(s) contain anomalies and, if so, can optionally identify anomalous elements within the file(s). Similarly, payload classification system 200 may execute a malware detection model that determines whether the file(s) contain malware and, if so, can optionally identify malicious elements within the file(s).

If the file(s) are determined not to contain anomalies or malware, payload classification system 200 may continue processing the file(s) as normal - such as by saving the file(s) locally for quick retrieval and/or transmitting the file(s) or copies of the file(s) to backend server(s) 106 for storage. If one or more file(s) are determined to contain anomalies or malware, payload classification system 200 may execute a content disarm and reconstruction process, as mentioned above. In some cases, payload classification system 200 may first store a copy of the original file(s) on backend server(s) 106. After the content disarm and reconstruction process, payload classification system 200 may reevaluate the file(s) for anomalies or malware. If, upon reevaluation, the file(s) are determined to no longer contain anomalies and/or malware, the file(s) may continue to be processed as normal. Otherwise, payload classification system 200 may initiate corrective actions, such as flagging the file(s) for manual review. Further details of payload classification system 200 are discussed below.

While shown as a separate component of PACS 102, it should be appreciated that payload classification system 200 may be integrated into or hosted on edge server(s) 104 and/or backend server(s) 106. In other implementations, payload classification system 200 includes or is hosted on a separate computing device. In some implementations, payload classification system 200 is a cloud-based system/server hosted on a cloud computing device, such as a cloud server. Thus, in some such implementations, payload classification system 200 may be hosted externally to or remotely from the other components of PACS 102. For example, payload classification system 200 may be accessed via a suitable application programming interface (API). In some implementations, a version of payload classification system 200 may be executed on each of edge server(s) 104, such that anomaly and/or malware detection can be executed before medical imaging data is stored on backend server(s) 106 and/or retrieved for viewing by workstation(s) 120. It should therefore be appreciated that various implementations of payload classification system 200 within PACS 102 are contemplated herein, and that the specific implementation shown in FIG. 1 is not intended to be limiting.

### Payload Classification System

Referring now to FIG. 2, a detailed block diagram of payload classification system 200 is shown, according to some implementations. Payload classification system 200 is shown to include a processing circuit 202 that includes a processor 204 and a memory 210. Processor 204 can be a general-purpose processor, an application-specific integrated circuit (ASIC), one or more field programmable gate arrays (FPGAs), a group of processing components, or other suitable electronic processing structures. In some implementations, processor 204 is configured to execute program code stored on memory 210 to cause payload classification system 200 to perform one or more operations, as described below in greater detail. It will be appreciated that, in implementations where payload classification system 200 is part of another computing device, the components of payload classification system 200 may be shared with, or the same as, the host device. For example, if payload classification system 200 is implemented via one of edge server(s) 104, then payload classification system 200 may utilize the processing circuit, processor(s), and/or memory of the host edge server to perform the functions described herein.

Memory 210 can include one or more devices (e.g., memory units, memory devices, storage devices, etc.) for storing data and/or computer code for completing and/or facilitating the various processes described in the present disclosure. In some implementations, memory 210 includes tangible (e.g., non-transitory), computer-readable media that stores code or instructions executable by processor 204. Tangible, computer-readable media refers to any physical media that is capable of providing data that causes payload classification system 200 to operate in a particular fashion. Example tangible, computer-readable media may include, but is not limited to, volatile media, non-volatile media, removable media and non-removable media implemented in any method or technology for storage of information such as computer readable instructions, data structures, program modules or other data. Accordingly, memory 210 can include random access memory (RAM), read-only memory (ROM), hard drive storage, temporary storage, non-volatile memory, flash memory, optical memory, or any other suitable memory for storing software objects and/or computer instructions. Memory 210 can include database components, object code components, script components, or any other type of information structure for supporting the various activities and information structures described in the present disclosure. Memory 210 can be communicably connected to processor 204, such as via processing circuit 202, and can include computer code for executing (e.g., by processor 204) one or more processes described herein.

While shown as individual components, it will be appreciated that processor 204 and/or memory 210 can be implemented using a variety of different types and quantities of processors and memory. For example, processor 204 may represent a single processing device or multiple processing devices. Similarly, memory 210 may represent a single memory device or multiple memory devices. Additionally, in some implementations, payload classification system 200 may be implemented within a single computing device (e.g., one server, one housing, etc.). In other implementations, payload classification system 200 may be distributed across multiple servers or computers (e.g., that can exist in distributed locations). For example, payload classification system 200 may include multiple distributed computing devices (e.g., multiple processors and/or memory devices) in communication with each other that collaborate to perform operations. For example, but not by way of limitation, an application may be partitioned in such a way as to permit concurrent and/or parallel processing of the instructions of the application. Alternatively, the data processed by the application may be partitioned in such a way as to permit concurrent and/or parallel processing of different portions of a data set by the two or more computers.

Memory 210 is shown to include an anomaly detector 212 and a malware detector 214, which include classification models for detecting anomalies and malware, respectively. A "classification" model, as described herein, generally refers to a machine learning model that predicts a class label or classification/category based on input data. In the case of anomaly detection, anomaly detector 212 can include a classification model - also referred to as an "anomaly detection" model - that predicts whether an input medical imaging file "contains anomalies" or "does not contain anomalies." Similarly, with respect to malware detection, malware detector 214 can include a classification model - also referred to as a "malware detection" model - that predicts whether an input medical imaging file "contains malware" or "does not contain malware." In some implementations, the classification models included in (e.g., implemented by) anomaly detector 212 and malware detector 214 are multi-layer perceptrons (MLPs), support vector machines (SVMs), random forest models, or convolutional neural networks (CNNs); although it should be appreciated that the present description is not limited to only these types of classification models. In some implementations, anomaly detector 212 and malware detector 214 utilize different versions of the same type of model. In other implementations, anomaly detector 212 and malware detector 214 utilize different types of models.

In some implementations, the classification model of anomaly detector 212 is trained for anomaly detection using a first training data set from a database 220. This "anomaly" training data set generally includes a plurality of known-good medical images and/or medical imaging files from medical imaging device(s) 110. In some implementations, the anomaly training data set is constructed from data provided by a variety of different imaging modalities and/or with a variety of attachments, embedded objects, and the like. In this regard, the classification model of anomaly detector 212 is trained to recognize valid or "good" medical imaging data from medical imaging device(s) 110 and, conversely, can identify data that is "out-of-place" or anomalous. For example, the anomaly training data set may include a plurality of files collected over time from multiple different medical imaging device(s) 110 associated with a particular medical facility or entity. Thus, by training the classification model of anomaly detector 212 using this known-good imaging data, anomaly detector 212 can identify anomalies in subsequent imaging data.

In some implementations, the classification model of anomaly detector 212 is trained using a supervised training technique; although, other suitable training techniques can be used. As will be appreciated, training a classification model generally includes providing the training data set - in this case, a robust set of known-good or valid medical imaging data collected from a plurality of imaging devices - as an input to the model. The model then generates a predicted classification for the input data, which is compared to known or existing labels. Model parameters can then be adjusted to align the predicted classifications with the known or actual classifications for each input medical imaging file. In some implementations, the model parameters are weights and/or biases.

In some implementations, the anomaly training data set is generated from a collection of medical imaging files collected over time from medical imaging device(s) 110. For example, an archived collection of medical imaging files stored on backend server(s) 106 and/or payload classification system 200 can be used as the training data set. In some such implementations, the anomaly training data set is generated by anomaly detector 212 itself. In some implementations, the classification model of anomaly detector 212 is trained on a data set that is specific to a particular entity (e.g., a medical facility, a group of medical facilities, etc.) to identify anomalies with respect to the medical imaging device(s) associated with that entity. In some implementations, anomaly detector 212 updates or augments the training data set as new medical imaging files are received and confirmed not to contain anomalies. In some implementations, the classification model of anomaly detector 212 is periodically or continuously updated/retrained as new medical imaging files are received and predicted not to contain anomalies. In this manner, the classification model of anomaly detector 212 can account for slight variations in medical imaging files, e.g., due to new imaging modalities.

Similarly, the classification model executed by malware detector 214 can be trained for malware detection using a second "malware" training data set, which generally includes a plurality of known-malicious files, data elements (e.g., code), and/or malware. In this manner, the classification model of malware detector 214 is trained to recognize malicious data or malware in medical imaging files. In some implementations, this malware training data set is maintained in database 220; however, more commonly, malware training data is maintained by a remote computing device (e.g., backend server(s) 106) for security reasons. For example, backend server(s) 106 may maintain, within a secure environment, a malware training database, which is used to train malware detector 214. In this manner, potentially malicious files are not communicated between devices. In some implementations, the classification model of malware detector 214 is trained using a supervised training technique, similar to the technique described above with respect to anomaly detector 212; although, other suitable training techniques can be used.

In some implementations, one or both of the classification models of anomaly detector 212 and malware detector 214 are trained locally, by anomaly detector 212 and malware detector 214, respectively. In other implementations, one or both of the classification models of anomaly detector 212 and malware detector 214 are trained remotely. In some such implementations, the remote device (e.g., backend server(s) 106) may train the classification model(s) locally and then transmit the trained models to payload classification system 200. For example, backend server(s) 106 may be configured to locally train anomaly and malware-detecting classification models, which are then distributed to edge server(s) 104 for use. In another example, anomaly detector 212 may train its classification model locally using data specific to an associated set of medical imaging device(s) 110, while the classification model of malware detector 214 is trained remotely and then transmitted to payload classification system 200. In this manner, anomaly detector 212 can accurately identify anomalies respective to the specific medical imaging devices from which it receives data, while malware detector 214 can accurately detect malware in said data. In some implementations, the models are trained periodically (e.g., every day, every week, etc.). In some implementations, the models are trained when a specific amount of new medical imaging data or malware data is collected. In some implementations, the models are trained initially by a remote device (e.g., backend server(s) 106) and then retrained or updated by payload classification system 200.

After the classification models are trained, anomaly detector 212 and malware detector 214 can be used to evaluate newly obtained medical imaging data (e.g., DICOM files). When a medical imaging file is obtained - either from a database or directly from one of medical imaging device(s) 110 - it can be fed into the classification models of anomaly detector 212 and malware detector 214 to generate an output of a predicted classification and a confidence score, or a classification score. In some implementations, a newly obtained medical imaging file is first evaluated by anomaly detector 212 and is only evaluated by malware detector 214 if anomalies are detected (e.g., as anomalies can be benign or malicious). In other implementations, a newly obtained medical imaging file is evaluated by both anomaly detector 212 and malware detector 214 regardless of whether anomalies are detected. In some such implementations, the medical imaging file is evaluated simultaneously by anomaly detector 212 and malware detector 214.

As will be appreciated by those of ordinary skill in the art, medical imaging files generally contain both metadata and images relating to a study. For example, DICOM files typically include a header that contains a preamble and a prefix, a series of standard and non-standard/custom text fields, and a data set that contains a number of data elements (e.g., images, documents). Notably, anomaly detector 212 and malware detector 214 are configured to evaluate both header data and payload data, whereas most existing malware detection solutions are not able to evaluate metadata, images, and/or embedded documents. In some implementations, anomaly detector 212 and/or malware detector 214 evaluate header and payload data simultaneously. In other implementations, header and payload data is evaluated separately. With respect to malware detection, malware detector 214 may implement a moving window to evaluate the file payload (e.g., image data), as malware is typically "small." In some implementations, anomaly detector 212 may also use a moving window to evaluate image data.

In some implementations, prior to or as part of evaluating a medical imaging file, anomaly detector 212 and/or malware detector 214 are configured to convert images within the file into greyscale. Specifically, in some implementations, images (e.g., the medical imaging file payload) may be converted into a plurality of unsigned 8-bit integers ranging from 0-255. Anomaly detector 212 and/or malware detector 214 may then evaluate the converted images, as part of evaluating the entire medical imaging file, to identify anomalies and/or malware that is embedded in the image(s). To this point, the classification models of anomaly detector 212 and/or malware detector 214 may be trained using greyscale-converted images such that, when new medical images are received and converted, the new images can be evaluated using trained models to identify valid features and/or patterns (e.g., in the case of anomaly detection) and/or malicious data (e.g., in the case of malware detection).

In some implementations, each of anomaly detector 212 and malware detector 214 are configured to determine (e.g., using their respective classification models) a score that is indicative of a likelihood that the input medical imaging data contains anomalies (e.g., "is anomalous") or malware (e.g., "is malicious"). This score is sometimes referred to as a probability score or a confidence that the input data fits a given class (e.g., "contains malware" or "is malicious"). Using anomaly detector 212 as an example, the classification model may output (e.g., for a given medical imaging file) a score indicative of a probability that the input file contains anomalies. Typically, this value is between '0' and ` 1', but the probability score can also be represented as a percentage or a value out of 100. For example, if the classification model outputs a value of 0.7, it may be likely (e.g., there is a 70% probability) that the file contains anomalies; whereas, a value of 0.3 may indicate that it is unlikely the file contains anomalies.

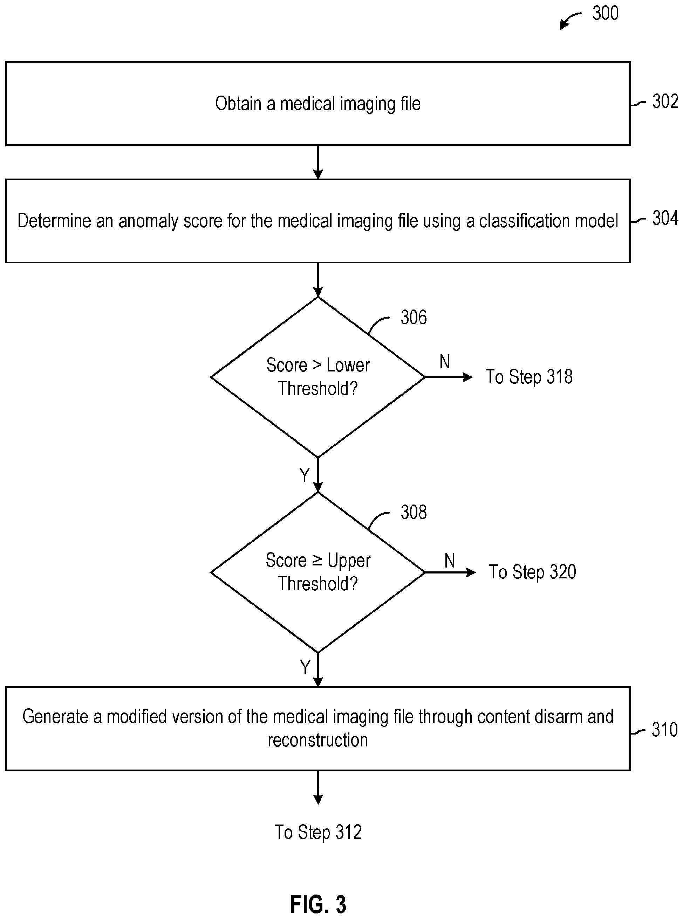

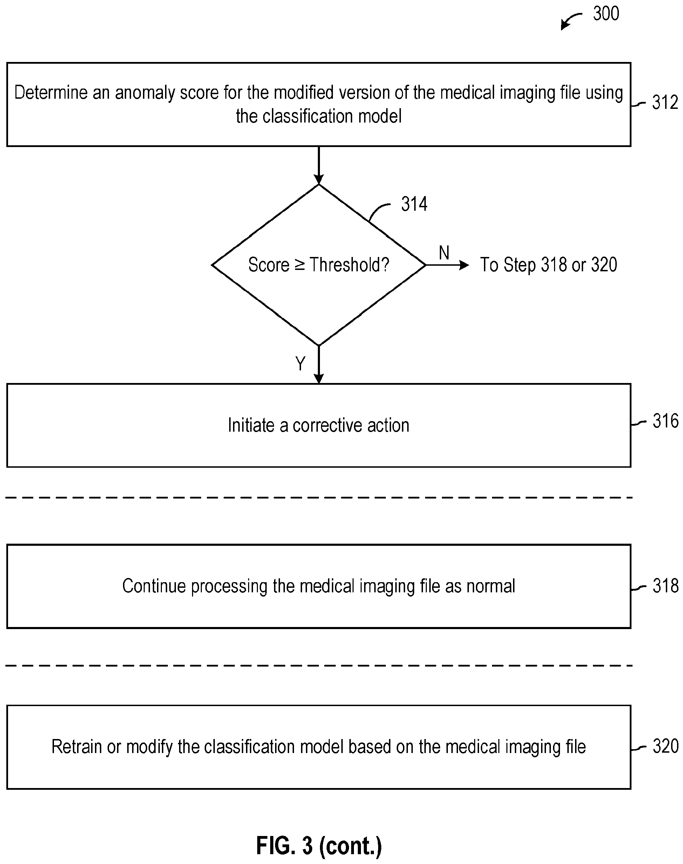

In some implementations, as described in greater detail below with respect to FIG. 3, one or more threshold values can be established to assign a label of "contains anomalies" or "does not contain anomalies" to a given file. For example, the threshold may be a score of 0.5 where, if a file is scored above 0.5, it is deemed as containing anomalies. However, if the score is below 0.5, in this example, the file may be deemed free of anomalies. In some implementations, anomaly detector 212 and malware detector 214 can compare the scores provided by their respective classification models to predefined thresholds to assign a label to each file. In some implementations, rather than outputting "scores", anomaly detector 212 can output a classification of either "contains anomalies" or "does not contain anomalies" and malware detector 214 can output a classification of either "contains malware" or "does not contain malware." Alternatively, anomaly detector 212 can classify an input as "okay" or "anomaly" and malware detector 214 can classify an input as "okay" or "malware."

In some implementations, based on the results of evaluating a medical imaging file, anomaly detector 212 and/or malware detector 214 can initiate corrective actions. For example, if a file is determined to contain anomalies, anomaly detector 212 may initiate corrective actions to confirm the prediction and/or to correct or remove the anomalies. In some implementations, anomaly detector 212 and/or malware detector 214 can utilize a content disarm and reconstruction (CDR) subsystem 216 as part of said corrective actions. As is its namesake, CDR subsystem 216 is generally configured to implement content disarm and reconstruction techniques on medical imaging files that are determined to contain anomalies and/or malware. In some implementations, CDR subsystem 216 disassembles the file, removes any data elements that are suspected of being anomalous and/or malicious, and reconstructions or reassembles the file in a secure environment (e.g., without the anomalous and/or malicious data elements). Put another way, CDR subsystem 216 can be thought of as "sanitizing" the medical imaging file from any suspected anomalous and/or malicious data. Notably, evaluating the file(s) for anomalies and/or malware before content disarm and reconstruction not only improves processing speeds but also reduces computational loads for local or edge devices. Put another way, content disarm and reconstruction can be a computationally expensive process and introduced processing delays; thus, performing anomaly and/or malware detection prior to content disarm and reconstruction - rather than performing content disarm and reconstruction on all files - can save time and reduce computational overhead.

In some implementations, anomaly detector 212 and/or malware detector 214 can reevaluate a medical imaging file after being processed by CDR subsystem 216 to determine whether the suspected anomalous and/or malicious data is removed. For example, a file that is predicted to contain anomalies can be processed by CDR subsystem 216 to generate a modified or reconstructed file (e.g., having had any identified/suspect anomalous data elements removed) and the modified or reconstructed file can then be reevaluated by anomaly detector 212 to confirm whether the CDR process removed the anomalies. If so, the modified or reconstructed file may proceed with normal processing procedures (e.g., storage in backend server(s) 106). If not, then the file may be flagged for manual review and/or quarantined. Similarly, a predicted malicious file can be processed by CDR subsystem 216 and the modified or reconstructed file can then be reevaluated by malware detector 214 to confirm whether the CDR process removed malicious elements.

In some implementations, medical imaging files are stored in a database 218 after anomaly detector 212 and/or malware detector 214 determine that the file(s) are anomaly and/or malware-free. In some implementations, database 218 further stores medical imaging files prior to evaluation. In some such implementations, unevaluated files are stored in a secure portion of database 218 or are otherwise portioned from evaluated files. In some implementations, anomaly and/or malware-free medical imaging files are stored in database 218 for retrieval and/or viewing by workstation(s) 120 or other computing device, as mentioned above. In some such implementations, medical imaging files are stored in database 218 for a limited period of time (e.g., a day, a week, a month) before being transferred to backend server(s) 106 for archiving. In some implementations, medical imaging files are stored in database 218 until a storage limit is reached, after which time the oldest files are incrementally transferred to backend server(s) 106 for archiving.

Still referring to FIG. 2, payload classification system 200 is also shown to include a communications interface 230 that facilitates communications between payload classification system 200 and any external components or devices, such as medical imaging device(s) 110, workstation(s) 120, and other components of PACS 102. Accordingly, communications interface 230 can be or can include a wired or wireless communications interface (e.g., jacks, antennas, transmitters, receivers, transceivers, wire terminals, etc.) for conducting data communications, or a combination of wired and wireless communication interfaces. In some implementations, communications via communications interface 230 are direct (e.g., local wired or wireless communications) or via a network (e.g., a WAN, the Internet, a cellular network, etc.). For example, communications interface 230 may include one or more Ethernet ports for communicably coupling payload classification system 200 to a network (e.g., the Internet). In another example, communications interface 230 can include a Wi-Fi transceiver for communicating via a wireless communications network. In yet another example, communications interface 230 may include cellular or mobile phone communications transceivers. It should therefore be appreciated that the description of communications interface 230 provided herein is not intended to be limiting; rather, any suitable communication components (e.g., transceivers, jacks, terminals, etc.) for wired or wireless communications are contemplated herein.

### Anomaly Detection

As mentioned above, not all anomalies in medical imaging files are malicious in nature. For example, new imaging modalities may introduce harmless anomalies by generating payloads in new or different manners from existing imaging modalities. Further, many medical imaging files do not contain anomalies. To these points, the above-mentioned classification model executed by anomaly detector 212 can be used to quickly identify anomalous files, such that non-anomalous files can be more quickly processed and stored and thus are more quickly retrievable by end users via workstation(s) 120. At the same time, anomalous files can be further investigated to determine whether anomalies are harmful or benign. This anomaly detection process is described in greater detail below with respect to FIG. 3.

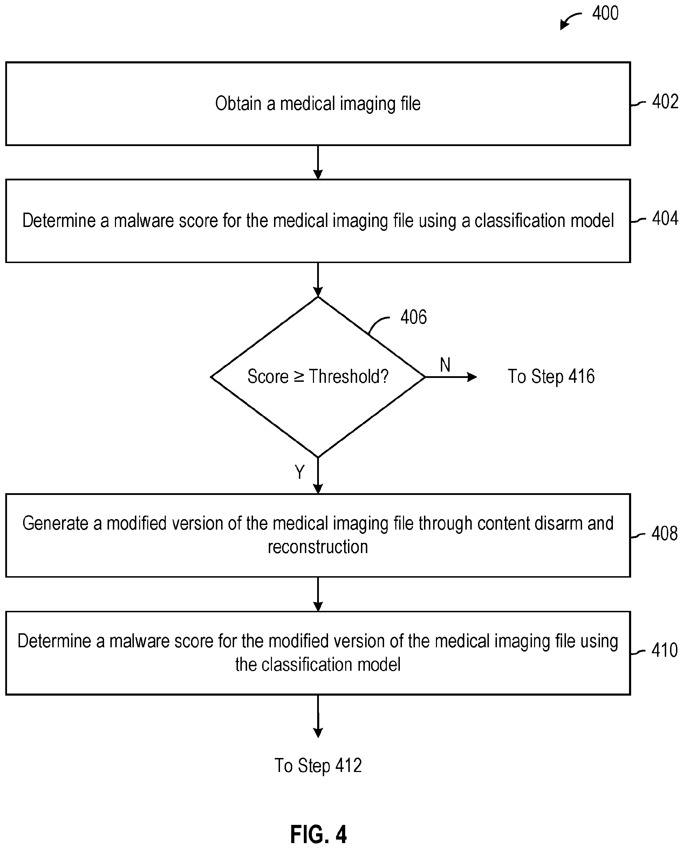

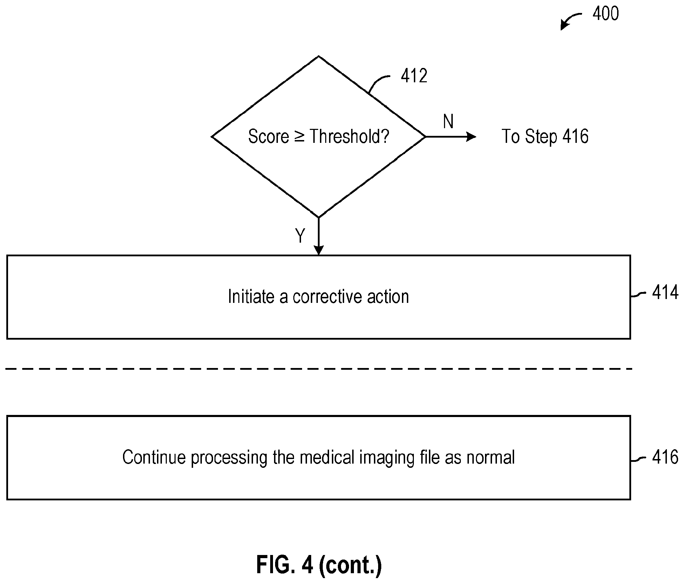

Consider, for example, a hospital that begins using a new type of MRI scanner that generates DICOM files in a different manner from the hospital's existing machines. Payload classification system 200 may determine that these new DICOM files contain anomalies because they do not match historical imaging data from the hospital's existing machines. However, rather than assuming the new DICOM files are malicious, payload classification system 200 may determine that the detected anomalies are harmless and therefore are likely caused by the new imaging modality. Alternatively, payload classification system 200 can further evaluate the new DICOM files using the above-mentioned malware detection techniques (e.g., also described below with respect to FIG. 4) to determine whether the identified anomalies are malicious.

Referring now to FIG. 3, a flow chart of a process 300 for detecting anomalies in medical imaging data is shown, according to some implementations. In some implementations, process 300 is implemented by payload classification system 200, as described above. Specifically, in some implementations, process 300 is implemented by an edge server (e.g., one of edge server(s) 104) which hosts payload classification system 200. However, it should be appreciated that process 300 may be at least partially implemented by another device or system. It will be appreciated that certain steps of process 300 may be optional and, in some implementations, process 300 may be implemented using less than all of the steps. It will also be appreciated that the order of steps shown in FIG. 3 is not intended to be limiting.

At step 302, a medical imaging file is obtained. As discussed above, the medical imaging file may be a DICOM file, but it should be appreciated that other formats of medical imaging file are contemplated herein. Generally, the medical imaging file includes one or more images captured by a medical imaging device (e.g., an X-ray machine, an MRI, a CT scanner, etc.) and metadata associated with the images, the medical imaging device that captured the images, the patient associated with the images, and/or other aspects of the study. In some implementations, the medical imaging file is generated by the medical imaging device and transmitted to an edge server (e.g., one of edge server(s) 104); therefore, the medical imaging file is received by the edge server (e.g., on which payload classification system 200 is executing or is hosted). In some implementations, the medical imaging file is obtained (e.g., retrieved) from a database or archive of medical imaging files. In some implementations, the medical imaging file is received from another remote or third-party device (e.g., a gateway device, a computer, etc.).

At step 304, an anomaly score is determined for the medical imaging file using a trained classification model. In particular, the medical imaging file may be fed (e.g., as an input) into a classification model that is trained to identify anomalies. It should be appreciated that evaluating the medical imaging file using the classification model generally includes evaluating both the contained metadata (e.g., header) and image data. In some implementations, the metadata and image data are separated and separately processed by the classification model. In other implementations, the classification model is configured to process both metadata and image data. In some implementations, the output of the classification model (generated from the input medical imaging file) is a probability or confidence score - herein referred to as an "anomaly score" - that is indicative of a likelihood that the input medical imaging data contains anomalies. Put another way, the classification model can generate a score that represents a probability or confidence that the medical imaging file fits into a class associated with "containing anomalies." Alternatively, the classification model could be configured to determine a likelihood that the file does not contain anomalies.

As described above with respect to anomaly detector 212, the classification model can be, but is not limited to, one of an MLP, an SVM, a random forest model, a CNN, or another suitable type of classification model (e.g., a logistic regression model, a decision tree, etc.). In some implementations, the classification model is a type of artificial neural network (ANN). A convolutional neural network (CNN) is a type of deep neural network that has been applied, for example, for classification of input data. Unlike a traditional neural networks, each layer in a CNN has a plurality of nodes arranged in three dimensions (width, height, depth). CNNs can include different types of layers, e.g., convolutional, pooling, and fully-connected (also referred to herein as "dense") layers. A convolutional layer includes a set of filters and performs the bulk of the computations. A pooling layer is optionally inserted between convolutional layers to reduce the computational power and/or control overfitting (e.g., by downsampling). A fully-connected layer includes neurons, where each neuron is connected to all of the neurons in the previous layer. The layers are stacked similar to traditional neural networks. A support vector machine (SVM) is a supervised learning model that uses statistical learning frameworks to predict the probability of a target. This disclosure contemplates that the SVMs can be implemented using a computing device (e.g., a processing unit and memory as described herein). SVMs can be used for classification and regression tasks. SVMs are trained with a data set (also referred to herein as a "dataset") to maximize or minimize an objective function, for example a measure of the SVM's performance, during training. An ANN having hidden layers can also be referred to as deep neural network or multilayer perceptron (MLP).

In some implementations, the anomaly score is a value between '0' and '1' but can also be represented as a percentage or a value out of 100. In some implementations, the classification model generates multiple scores - one indicating a likelihood that the file contains anomalies and another indicating a likelihood that the file does not contain anomalies. In some implementations, rather than generating a "score," the medical imaging file is simply classified as either "contains anomalies" or "does not contain anomalies." Alternatively, the medical imaging file is classified as either "okay" or "anomalous." In some implementations, these classifications are determined by the classification model itself. In other implementations, anomaly detector 212 interprets an output of the model (e.g., a probability score) to determine the classification. In some implementations, the classification model outputs both a predicted classification (e.g., "anomalous") and a confidence score for the prediction.

As described herein, the anomaly detection classification model (e.g., the classification model of anomaly detector 212) is generally trained using a training data set of known-good medical imaging data. In some implementations, the known-good medical imaging data is collected from a set of medical imaging devices over time. In some such implementations, the training data set can be constructed from historical imaging data associated with a particular medical imaging device or a group of devices associated with a particular entity. For example, the training data set may be constructed from medical imaging data collected for a particular medical facility; therefore, the anomaly detection classification model can be trained to identify anomalies in medical imaging data received from the particular medical facility. For the sake of brevity, additional discussion of the training of the anomaly detection classification model is provided above with respect to FIG. 2. In some implementations, as also discussed above, prior to or as part of determining the anomaly score, image data within the medical imaging file is converted to greyscale. Specifically, in some implementations, images (e.g., the medical imaging file payload) may be converted into a plurality of unsigned 8-bit integers ranging from 0-255.

After determining the anomaly score using the classification model, the anomaly score can be compared to one or more thresholds, or a range of values, to determine whether the medical imaging file contains anomalies and/or if additional actions should be initiated. In some implementations, a range of values is defined by predefined lower and upper thresholds. In some such implementations - specifically, in implementations where the classification model generates an anomaly score indicative of a likelihood that a file contains anomalies - an anomaly score that is below the lower threshold is associated with a file that is predicted to be anomaly-free. On the other hand, an anomaly score that meets or exceeds an upper threshold may be deemed to contain significant anomalies. In some implementations, an anomaly score that falls between the lower and upper thresholds is indicative of a file that may contain harmless anomalies (e.g., due to a new imaging modality).

In some implementations, if the anomaly score is determined to be less than the lower threshold (step 306), the file may be deemed "anomaly free," in which case process 300 may continue to step 318. Otherwise, the anomaly score may be compared to an upper threshold (step 308). In some such implementations, if the anomaly score is less than the upper threshold but greater than the lower threshold (e.g., between the lower and upper thresholds), the file may be deemed as containing anomalies that are most likely benign and process 300 may continue to step 320. Steps 318 and 320 are described in greater detail below. In some implementations, if the anomaly score meets or exceeds the upper threshold, the file may be identified as containing anomalies that may be harmful or malicious, and process 300 may continue to step 310. Consider, for example, a medical imaging file that returns an anomaly score of 0.7 (e.g., indicating a 70% probability or confidence that the file contains anomalies). If the upper threshold is 0.6, then the file may be classified as containing significant anomalies. If the upper threshold is instead 0.8 and the lower threshold 0.6, the file may be classified as containing anomalies that are likely harmless.

While "anomaly scores" are generally described herein with respect to process 300, it should be appreciated that the anomaly detection classification model can directly classify a file as "contains anomalies" or "does not contain anomalies," or "okay" or "anomalous." For example, in some implementations, the anomaly detection classification model may output a classification and, optionally, a confidence score for the classification. In some such implementations, process 300 may proceed to one of steps 312, 318, or 320 from step 304 based on the classification. For example, if a file is classified as "okay," process 300 may proceed directly to step 318. Thus, it should be understood that process 300 is not limited to generating anomaly scores and comparing the anomaly scores to thresholds; rather, other methods of classifying medical imaging files are contemplated herein. It will also be appreciated that, in some implementations, the anomaly detection classification model is configured to generate a prediction of a likelihood that a file is anomaly-free. In some such implementations, a file that meets or exceeds the upper threshold may be deemed "anomaly-free," whereas a file that is below the lower threshold would be considered to "contain anomalies."

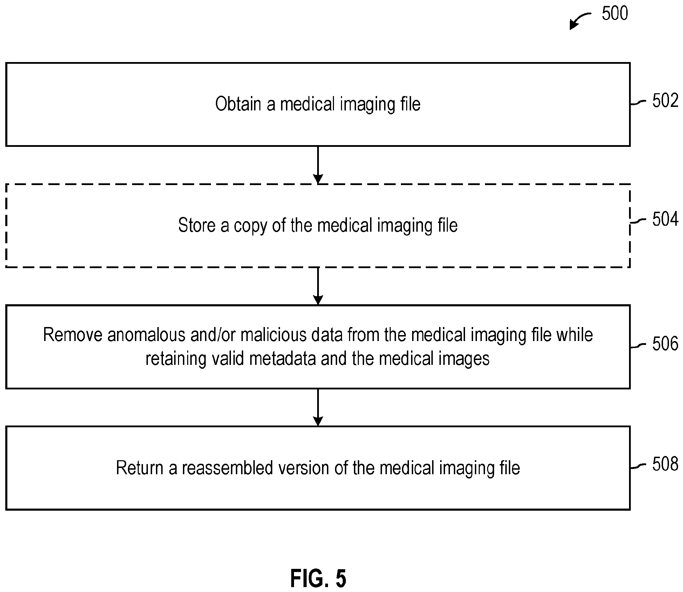

At step 310, a modified version of the medical imaging file is generated using a content disarm and reconstruction technique (e.g., if it is determined that the anomaly score meets or exceeds the upper threshold). Content disarm and reconstruction generally refers to a technique of disassembling, cleaning or "sanitizing," and the reassembling a file in a secure environment. With respect to a medical imaging file, data elements that are suspected of being anomalous can be removed prior to the medical imaging filing being reconstructed (e.g., modified). In some implementations, potentially anomalous data elements are identified by the classification model (e.g., at step 304) as part of the evaluation of the file. For example, the classification model may further output an indication of any data elements that negatively impacted the anomaly score. In some implementations, any non-essential data elements may be stripped from the medical imaging file as part of the content disarm and reconstruction process. In some implementations, any data that does not fit a predefined set of rules or parameters is removed. Additional details of the content disarm and reconstruction process are provided below with respect to FIG. 5.

At step 312, the modified (e.g., reconstructed) medical imaging file is reevaluated using the anomaly detection classification model to generate a new anomaly score. In this regard, step 312 is largely the same as, or substantially similar to, step 304 as described above. Generating an anomaly score for the modified medical imaging file can indicate whether the content disarm and reconstruction process successfully removed the anomalous data elements. For example, if the anomaly score for the modified medical imaging file is lower than the original anomaly score, it may be assumed that the content disarm and reconstruction process was successful in removing at least some of the anomalous elements. In some implementations, if the new anomaly score is less than a predefined threshold (step 314), the process 300 may proceed to one of steps 318 or 320, depending on the configuration of payload classification system 200. In some implementations, the threshold is the same as the upper threshold of step 308. In other implementations, the threshold is the same as the lower threshold of step 306. In yet other implementations, the threshold is a different value from the lower and upper thresholds. For example, the threshold considered at step 314 may be lower than the upper threshold of step 308 for a stricter classification the modified medical imaging file.

At step 316, corrective actions are initiated if it is determined that the new anomaly score (e.g., of the modified medical imaging file) meets or exceeds the threshold. In other words, corrective actions are initiated if the medical imaging file is determined to contain anomalies both initially and after content disarm and reconstruction. In some implementations, the corrective actions include flagging the medical imaging file for additional or manual review. For example, the medical imaging file may be appended with a flag or identifier indicating that further review is required. In some implementations, flagging the medical imaging file includes generating and/or displaying a notification (e.g., to a user of payload classification system 200 or one of workstation(s) 120) identifying the potentially anomalous file. For example, a notification may be displayed within a DICOM image viewing interface or transmitted to a remote computing device. In some implementations, the corrective actions include quarantining or otherwise separating the anomalous file from other, non-anomalous files. For example, the anomalous file may be placed in a quarantine folder or portioned memory section. In some implementations, the anomalous file is still available for viewing (e.g., on workstation(s) 120) from the quarantine folder; however, an alert may be presented to a user viewing the file and/or the file may be opened in a secure environment. In some implementations, the corrective actions include evaluating the medical imaging file for malware via the process described below with respect to FIG. 4.

At step 318 - continuing from step 306 - the medical imaging file is processed as normal. In particular, the medical imaging file may be processed as normal if the anomaly score of the original medical imaging file is determined to be less than the lower threshold, or the file is otherwise classified as "okay" or "does not contain anomalies." In some implementations, "normal" processing refers to any processing steps normally implemented by a PACS, as would be understood by one of ordinary skill in the art. For example, in some implementations, if a file is deemed anomaly-free, the file may be stored locally (e.g., on an edge server) and/or transmitted to a backend server for archiving. In some implementations, the modified medical imaging file is also processed according to normal procedures if its anomaly score is less than the threshold at step 314.

At step 320 - continuing from step 308 - the medical imaging file is optionally used to retrain or modify the anomaly detection classification model. In this regard, if the anomaly score for the medical imaging file is determined to be between the lower and upper thresholds, the medical imaging file may be classified as containing anomalies that are likely harmless or benign (e.g., due to new imaging modalities). Accordingly, the anomaly detection classification model may be retrained to account for new data elements, formats, or other characteristics associated with the new imaging modalities. In some implementations, the anomaly detection classification model is retrained locally (e.g., on an edge server). In some implementations, rather than the anomaly score being between the lower and upper thresholds, the classification model is retrained using the medical imaging file if the medical imaging file's anomaly score is within a predetermined range of a single (e.g., the upper) threshold. For example, if the threshold for anomalous/not anomalous is 0.7 and a file's anomaly score is 0.65, the file may be determined to be close enough to an acceptable (e.g., not anomalous) score that any anomalies are likely due to new imaging modalities.

### Malware Detection

Referring now to FIG. 4, a flow chart of a process 400 for detecting malware in medical imaging data is shown, according to some implementations. In some implementations, process 400 is executed separately from process 300, described above. For example, process 400 may be executed before, after, or concurrently with process 300. In some implementations, process 400 is executed if a file is determined to contain anomalies. To this point, process 400 may be a "corrective action" taken at step 316 of process 300. In some implementations, process 400 is implemented by payload classification system 200, as described above. However, it should be understood that process 400 may be at least partially implemented by another device or system. It will be appreciated that certain steps of process 400 may be optional and, in some implementations, process 400 may be implemented using less than all of the steps. It will also be appreciated that the order of steps shown in FIG. 4 is not intended to be limiting.

At step 402, a medical imaging file is obtained. As discussed above, the medical imaging file may be a DICOM file, but it should be appreciated that other formats of medical imaging file are contemplated herein. Generally, the medical imaging file includes one or more images captured by a medical imaging device (e.g., an X-ray machine, an MRI, a CT scanner, etc.) and metadata associated with the images, the medical imaging device that captured the images, the patient associated with the images, and/or other aspects of the study. In some implementations, the medical imaging file is generated by the medical imaging device and transmitted to an edge server (e.g., one of edge server(s) 104); therefore, the medical imaging file is received by the edge server (e.g., on which payload classification system 200 is executing or is hosted). In some implementations, the medical imaging file is obtained (e.g., retrieved) from a database or archive of medical imaging files. In some implementations, the medical imaging file is received from another remote or third-party device (e.g., a gateway device, a computer, etc.).

At step 404, a malware score is determined for the medical imaging file using a trained classification model. In particular, the medical imaging file may be fed (e.g., as an input) into a classification model that is trained to identify malware. It should be appreciated that evaluating the medical imaging file using the classification model generally includes evaluating both the contained metadata (e.g., header) and image data. In some implementations, the metadata and image data are separated and separately processed by the classification model. In other implementations, the classification model is configured to process both metadata and image data. As described above with respect to malware detector 214, the classification model can be, but is not limited to, one of an MLP, an SVM, a random forest model, a CNN, or another suitable type of classification model. In some implementations, the output of the classification model (generated from the input medical imaging file) is a probability or confidence score - herein referred to as a "malware score" - that is indicative of a likelihood that the input medical imaging data contains malware. Put another way, the classification model can generate a score that represents a probability or confidence that the medical imaging file fits into a class associated with "containing malware." Alternatively, the classification model could be configured to determine a likelihood that the file does not contain malware.

In some implementations, the malware score is a value between '0' and '1' but can also be represented as a percentage or a value out of 100. In some implementations, the classification model generates multiple scores - one indicating a likelihood that the file contains malware and another indicating a likelihood that the file does not contain malware. In some implementations, rather than generating a "score," the medical imaging file is simply classified as either "contains malware" or "does not contain malware." Alternatively, the medical imaging file is classified as either "okay" or "malicious." In some implementations, these classifications are determined by the classification model itself. In other implementations, malware detector 214 interprets an output of the model (e.g., a probability score) to determine the classification. In some implementations, the classification model outputs both a predicted classification (e.g., "malicious") and a confidence score for the prediction.

As described herein, the malware detection classification model (e.g., the classification model of malware detector 212) is generally trained using a training data set of known-malicious data or malware. In particular, the training data set can include or be constructed from a variety of malware samples. In this manner, the malware detection classification model can be trained to detect malware and/or to predict whether data is malicious based on known malware. In some implementations, the malware detection classification model is trained remotely and/or in a secure environment, to avoid the risk of unintentionally passing malware between devices. For example, the malware detection classification model may be trained on one of backend server(s) 106 and transmitted to payload classification system 200 operating on one of edge server(s) 104. For the sake of brevity, additional discussion of the training of the malware detection classification model is provided above with respect to FIG. 2.

At step 406, the malware score is compared to a threshold to determine whether the medical imaging file is possibly malicious, and therefore requires additional processing or analysis, or is likely not malicious. In some implementations, the threshold is a predetermined value that delineates "okay" files from "malicious" files. Specifically, in some implementations, malware scores that meet or exceed the threshold may be deemed as "likely containing" malware, whereas malware scores below the threshold are deemed as likely malware-free. For example, a medical imaging file that returns a malware score of 0.8 (e.g., indicating an 80% probability or confidence that the file contains malware) may be determined to "contain malware" if the threshold is 0.7.

While "malware scores" are generally described herein with respect to process 400, it should be appreciated that the malware detection classification model can directly classify a file as "contains malware" or "does not contain malware," or "okay" or "malicious." For example, in some implementations, the malware detection classification model may output a classification and, optionally, a confidence score for the classification. In some such implementations, process 400 may proceed directly to step 416 from step 404 based on the classification. For example, if a file is classified as "okay," process 400 may proceed directly to step 416. Thus, it should be understood that process 400 is not limited to generating malware scores and comparing the malware scores to thresholds; rather, other methods of classifying medical imaging files are contemplated herein. It will also be appreciated that, in some implementations, the malware detection classification model is configured to generate a prediction of a likelihood that a file is malware-free. In some such implementations, a file that meets or exceeds the threshold may be deemed "malware-free" whereas a file that is below the lower threshold would be considered to "contain malware."

At step 408, a modified version of the medical imaging file is generated using a content disarm and reconstruction technique (e.g., if it is determined that the malware score meets or exceeds the upper threshold). With respect to a medical imaging file, data elements that are suspected of being malicious can be removed prior to the medical imaging filing being reconstructed (e.g., modified). In some implementations, potentially malicious data elements are identified by the classification model (e.g., at step 404) as part of the evaluation of the file. For example, the classification model may further output an indication of any data elements that negatively impacted the malicious score. In some implementations, any non-essential data elements may be stripped from the medical imaging file as part of the content disarm and reconstruction process. In some implementations, any data that does not fit a predefined set of rules or parameters is removed. Additional details of the content disarm and reconstruction process are provided below with respect to FIG. 5.

At step 410, the modified (e.g., reconstructed) medical imaging file is reevaluated using the malware detection classification model to generate a new malware score. In this regard, step 410 is largely the same as, or substantially similar to, step 404 as described above. Generating a malware score for the modified medical imaging file can indicate whether the content disarm and reconstruction process successfully removed the malicious data elements. For example, if the malware score for the modified medical imaging file is lower than the original malware score, it may be assumed that the content disarm and reconstruction process was successful in removing at least some of the malicious elements. In some implementations, if the new malware score is less than a threshold (step 412), process 400 may proceed to step 416. In some implementations, the threshold is the same as the threshold of step 406. In other implementations, the threshold is a different value from the thresholds of step 406.

At step 414, corrective actions are initiated if it is determined that the new malware score (e.g., of the modified medical imaging file) meets or exceeds the threshold. In other words, corrective actions are initiated if the medical imaging file is determined to contain malware both initially and after content disarm and reconstruction. In some implementations, the corrective actions include flagging the medical imaging file for additional or manual review. For example, the medical imaging file may be appended with a flag or identifier indicating that further review is required. In some implementations, flagging the medical imaging file includes generating and/or displaying a notification (e.g., to a user of payload classification system 200 or one of workstation(s) 120) identifying the potentially malicious file. For example, a notification may be displayed within a DICOM image viewing interface or transmitted to a remote computing device. In some implementations, the corrective actions include quarantining or otherwise separating the malicious file from other, non-malicious files. For example, the anomalous file may be placed in a quarantine folder or portioned memory section. In some implementations, the malicious file is still available for viewing (e.g., on workstation(s) 120) from the quarantine folder; however, an alert may be presented to a user viewing the file and/or the file may be opened in a secure environment.

At step 416 - continuing from step 406 and/or 412 - the medical imaging file is processed as normal. In particular, the medical imaging file may be processed as normal if the malware score of the original medical imaging file is determined to be less than the threshold, or the file is otherwise classified as "okay" or "does not contain malware." In some implementations, "normal" processing refers to any processing steps normally implemented by a PACS, as would be understood by one of ordinary skill in the art. For example, in some implementations, if a file is deemed malware-free, the file may be stored locally (e.g., on an edge server) and/or transmitted to a backend server for archiving. In some implementations, the modified medical imaging file is also processed according to normal procedures if its malware score is less than the threshold at step 412.

### Content Disarm and Reconstruction

Referring now to FIG. 5, a flow chart of a process 500 for content disarm and reconstruction of medical imaging data is shown, according to some implementations. As described above, in various implementations, process 500 is executed as part of processes 300 and/or 400. Additionally, or alternatively, process 500 may be executed outside of processes 300 and 400. In some implementations, process 500 is implemented by payload classification system 200, as described above. However, it should be understood that process 500 may be at least partially implemented by another device or system. It will be appreciated that certain steps of process 500 may be optional and, in some implementations, process 500 may be implemented using less than all of the steps. It will also be appreciated that the order of steps shown in FIG. 5 is not intended to be limiting.

At step 502 a medical imaging file is obtained. As discussed above, the medical imaging file may be a DICOM file, but it should be appreciated that other formats of medical imaging file are contemplated herein. Generally, the medical imaging file includes one or more images captured by a medical imaging device (e.g., an X-ray machine, an MRI, a CT scanner, etc.) and metadata associated with the images, the medical imaging device that captured the images, the patient associated with the images, and/or other aspects of the study. In some implementations, the medical imaging file is generated by the medical imaging device and transmitted to an edge server (e.g., one of edge server(s) 104); therefore, the medical imaging file is received by the edge server (e.g., on which payload classification system 200 is executing or is hosted). In some implementations, the medical imaging file is obtained (e.g., retrieved) from a database or archive of medical imaging files. In some implementations, the medical imaging file is received from another remote or third-party device (e.g., a gateway device, a computer, etc.).

At step 504, a copy of the medical imaging file is optionally stored, e.g., for liability reasons. In some implementations, in particular, a copy of the medical imaging file is transmitted to and/or stored on one of backend server(s) 106. In other implementations, the copy of the medical imaging file is stored on another computing device. In this manner, an original version of the medical imaging file is retained in the event that the content disarm and reconstruction process (e.g., process 500) damages or corrupts the file. Additionally, the copy may serve as a reference to ensure that the content disarm and reconstruction process (e.g., process 500) does not alter crucial data within the file.

At step 506, anomalous and/or malicious data elements within the medical imaging file are removed, while valid metadata and images are retained. In other words, content disarm and reconstruction is performed on the medical imaging file to remove any anomalous and/or malicious data elements. In some implementations, content disarm and reconstruction includes disassembling the medical imaging file (e.g., into parts), removing any suspected anomalous and/or malicious data elements, and reassembling/reconstructing that medical imaging file in a secure environment. In some implementations, the suspected anomalous and/or malicious data elements are identified by the above-mentioned anomaly or malware detection classification models, e.g., as part of processes 300 and/or 400. In some implementations, step 506 includes identifying potentially anomalous or malicious data elements for removal. In some implementations, any out-of-the-ordinary data elements are removed (e.g., without consideration for whether the elements are anomalous or malicious). In some such implementations, any data elements that do not fit a predefined set of parameters or a template are removed. In some implementations, any data elements that are not within a predefined set of data elements are removed. In some implementations, any extraneous data from the preamble of the medical imaging file (e.g., embedded files, script, code, links, macros, etc.) are removed.

At step 508, a reassembled version of the medical imaging file is returned. In particular, after removing the anomalous or malicious data elements, the reassembled version of the medical imaging file may be reevaluated as described above with respect to process 300 and/or 400. In this regard, the reassembled version of the medical imaging file may be evaluated to determine whether the content disarm and reconstruction process (e.g., process 500) was successful in removing anomalous and/or malicious data. In some implementations, the reassembled version of the medical imaging file is stored in a database for later evaluation.

### Configuration of Certain Implementations

The construction and arrangement of the systems and methods as shown in the various implementations are illustrative only. Although only a few implementations have been described in detail in this disclosure, many modifications are possible (e.g., variations in sizes, dimensions, structures, shapes and proportions of the various elements, values of parameters, mounting arrangements, use of materials, colors, orientations, etc.). For example, the position of elements may be reversed or otherwise varied, and the nature or number of discrete elements or positions may be altered or varied. Accordingly, all such modifications are intended to be included within the scope of the present disclosure. The order or sequence of any process or method steps may be varied or re-sequenced according to alternative implementations. Other substitutions, modifications, changes, and omissions may be made in the design, operating conditions, and arrangement of the implementations without departing from the scope of the present disclosure.

The present disclosure contemplates methods, systems, and program products on any machine-readable media for accomplishing various operations. The implementations of the present disclosure may be implemented using existing computer processors, or by a special purpose computer processor for an appropriate system, incorporated for this or another purpose, or by a hardwired system. Implementations within the scope of the present disclosure include program products including machine-readable media for carrying or having machine-executable instructions or data structures stored thereon. Such machine-readable media can be any available media that can be accessed by a general purpose or special purpose computer or other machine with a processor. By way of example, such machine-readable media can comprise RAM, ROM, EPROM, EEPROM, CD-ROM or other optical disk storage, magnetic disk storage or other magnetic storage devices, or any other medium which can be used to carry or store desired program code in the form of machine-executable instructions or data structures, and which can be accessed by a general purpose or special purpose computer or other machine with a processor.

When information is transferred or provided over a network or another communications connection (either hardwired, wireless, or a combination of hardwired or wireless) to a machine, the machine properly views the connection as a machine-readable medium. Thus, any such connection is properly termed a machine-readable medium. Combinations of the above are also included within the scope of machine-readable media. Machine-executable instructions include, for example, instructions and data which cause a general-purpose computer, special purpose computer, or special purpose processing machines to perform a certain function or group of functions.

Although the figures show a specific order of method steps, the order of the steps may differ from what is depicted. Also, two or more steps may be performed concurrently or with partial concurrence. Such variation will depend on the software and hardware systems chosen and on designer choice. All such variations are within the scope of the disclosure. Likewise, software implementations could be accomplished with standard programming techniques with rule-based logic and other logic to accomplish the various connection steps, processing steps, comparison steps and decision steps.

It is to be understood that the methods and systems are not limited to specific synthetic methods, specific components, or to particular compositions. It is also to be understood that the terminology used herein is for the purpose of describing particular implementations only and is not intended to be limiting.

As used in the specification and the appended claims, the singular forms "a," "an" and "the" include plural referents unless the context clearly dictates otherwise. Ranges may be expressed herein as from "about" one particular value, and/or to "about" another particular value. When such a range is expressed, another implementation includes from the one particular value and/or to the other particular value. Similarly, when values are expressed as approximations, by use of the antecedent "about," it will be understood that the particular value forms another implementation. It will be further understood that the endpoints of each of the ranges are significant both in relation to the other endpoint, and independently of the other endpoint.

"Optional" or "optionally" means that the subsequently described event or circumstance may or may not occur, and that the description includes instances where said event or circumstance occurs and instances where it does not.

Throughout the description and claims of this specification, the word "comprise" and variations of the word, such as "comprising" and "comprises," means "including but not limited to," and is not intended to exclude, for example, other additives, components, integers or steps. "Exemplary" means "an example of' and is not intended to convey an indication of a preferred or ideal implementation. "Such as" is not used in a restrictive sense, but for explanatory purposes.

Disclosed are components that can be used to perform the disclosed methods and systems. These and other components are disclosed herein, and it is understood that when combinations, subsets, interactions, groups, etc. of these components are disclosed that while specific reference of each various individual and collective combinations and permutation of these may not be explicitly disclosed, each is specifically contemplated and described herein, for all methods and systems. This applies to all aspects of this application including, but not limited to, steps in disclosed methods. Thus, if there are a variety of additional steps that can be performed it is understood that each of these additional steps can be performed with any specific implementation or combination of implementations of the disclosed methods.

## Claims

1. A system for detecting anomalies and malware in medical imaging data, the system comprising:
a processor; and
memory having instructions stored thereon that, when executed by the processor, cause the system to:
obtain a medical imaging file comprising a header and a data set, wherein the header includes metadata associated with the medical imaging file and the data set includes one or more images captured by a medical imaging device;
evaluate the medical imaging file using a classification model to: i) generate a first score representative of a likelihood that the medical imaging file contains anomalous or malicious data, and ii) identify suspected anomalous or malicious data within the medical imaging file;
if the first score meets or exceeds a first threshold, modify the medical imaging file by removing the suspected anomalous or malicious data from the medical imaging file while retaining valid metadata in the header and the one or more images in the data set; and
if the first score is less than the first threshold, at least one of: i) store the medical imaging file without said modification, or ii) retrain the classification model based on the medical imaging file.

2. The system of claim 1, the instructions further causing the system to:
evaluate the modified medical imaging file using the classification model to generate a second score representative of a likelihood that the modified medical imaging file contains anomalous or malicious data; and
if the second score meets or exceeds the first threshold, quarantine the medical imaging file or flag the medical imaging file for additional review.

3. The system of claim 1 or 2, wherein the medical imaging file is stored without modification if the first score is less than a second threshold, wherein the second threshold is lower than the first threshold.

4. The system of any of claims 1-3, wherein the medical imaging file is used to retrain the classification model if the first score is between the first threshold and a second threshold, wherein the second threshold is lower than the first threshold.

5. The system of any of claims 1-4, wherein the classification model is trained by a remote computing device, the instructions further causing the system to receive the classification model from the remote device after training.

6. The system of any of claims 1-5, wherein the processor and the memory are components of an edge server of a picture archiving and communication system (PACS), and wherein the medical imaging file is received by the edge server from the medical imaging device.

7. The system of any of claims 1-6, the instructions further causing the system to convert the images in the data set of the medical imaging file to greyscale prior to evaluating the medical imaging file using the classification model.

8. The system of any of claims 1-7, wherein the classification model is a first classification model and the first score is representative of a likelihood that the medical imaging file contains an anomaly, the instructions further causing the system to evaluate the medical imaging file using a second classification model that generates a second score representative of a likelihood that the medical imaging file contains malware.

9. A method for detecting anomalies and malware in medical imaging data, the method comprising:
obtaining a medical imaging file comprising a header and a data set, wherein the header includes metadata associated with the medical imaging file and the data set includes images captured by a medical imaging device;
evaluating the medical imaging file using a classification model to: i) determine a first score representative of a likelihood that the medical imaging file contains anomalous or malicious data, and ii) identify suspected anomalous or malicious data within the medical imaging file;
if the first score meets or exceeds a first threshold, modifying the medical imaging file by removing the suspected anomalous or malicious data from the medical imaging file while retaining valid metadata in the header and the images in the data set; and
if the first score is less than the first threshold, at least one of: i) storing the medical imaging file without said modification, or ii) retraining the classification model based on the medical imaging file.

10. The method of claim 9, further comprising:
evaluating the modified medical imaging file using the classification model to generate a second score representative of a likelihood that the modified medical imaging file contains anomalous or malicious data; and
if the second score meets or exceeds the first threshold, quarantining the medical imaging file or flagging the medical imaging file for additional review.

11. The method of claim 9 or 10, wherein the medical imaging file is stored without modification if the first score is less than a second threshold, wherein the second threshold is lower than the first threshold.

12. The method of any of claims 9-11, wherein the medical imaging file is used to retrain the classification model if the first score is between the first threshold and a second threshold, wherein the second threshold is lower than the first threshold.

13. The method of any of claims 9-12, wherein the classification model is trained by a remote computing device, the method further comprising receiving the classification model from the remote device after training.

14. The method of any of claims 9-13, wherein the medical imaging file is obtained by an edge server of a picture archiving and communication system (PACS) and from the medical imaging device.

15. The method of any of claims 9-14, further comprising converting the images in the data set of the medical imaging file to greyscale prior to evaluating the medical imaging file using the classification model.
